# FASCICULE DE BREVET EUROPEEN

(11) **EP 1 893 141 B1**
(45) Date de publication et mention de la délivrance du brevet: **01.10.2008**
(21) Numéro de dépôt: 05775775.9
(22) Date de dépôt: 01.06.2005
(51) Int. Cl.: A61F 5/00

(54) **Ballon intra-gastrique avec une valve à double membrane et kit de mise en place corréspondant**
Intragastrischer Ballon mit doppeltem Membranventil und dazugehöriges Einsetzungskit
Intra-gastric ballon with double membrane valve and corresponding kit for setting the same

(43) Date de publication de la demande: 05.03.2008
(73) Titulaire: Compagnie Européenne d'Etude et de Recherche de Dispositifs pour l'Implantation par Laparoscopie, 38200 Vienne (FR)
(72) Inventeur: VALENCON, Nicolas, François, Michel, F-38780 Pont Eveque (US); PAGANON, Pascal, F-69360 Serezin du Rhône (FR)
(74) Mandataire: Martin, Didier Roland Valéry
(86) Numéro de dépôt international: PCT/FR2005/001343
(87) Numéro de publication internationale: WO 2006/128978

(56) Documents cités:
- FR-A- 2 685 751
- US-A- 2 151 466
- US-A- 4 263 682
- US-A- 4 739 758
- US-A- 5 456 716

## Description

### DOMAINE TECHNIQUE

La présente invention se rapporte au domaine technique général des dispositifs implantables dans le corps humain destinés à être utilisés dans le cadre d'un traitement de l'obésité et notamment de l'obésité morbide, et tout particulièrement à des implants aptes à réduire artificiellement le volume de l'estomac en vue notamment de produire une sensation de satiété chez le patient.

La présente invention concerne plus particulièrement un ballon intra gastrique destiné à être implanté à l'intérieur de l'estomac d'un patient pour réduire le volume de l'estomac dans le cadre d'un traitement de l'obésité, ledit ballon comprenant :
- au moins une poche apte à être remplie, au moins partiellement, avec un fluide de gonflage,
- un moyen de connexion, monté sur ladite poche et comportant un organe auto-obturant apte à être transpercé par un organe de connexion à une source de fluide de gonflage.

La présente invention concerne également un kit de mise en forme d'un ballon intra-gastrique destiné à être implanté au sein de l'estomac d'un patient dans le cadre d'un traitement de l'obésité comprenant :
- un ballon intra-gastrique,
- un organe de connexion du ballon à une source de fluide de gonflage.

### TECHNIQUE ANTERIEURE

Pour traiter les patients atteints d'obésité, il est connu d'utiliser des ballons intra-gastriques destinés à être implantés dans l'estomac des patients pour diminuer l'espace disponible pour les aliments, en vue notamment de produire une sensation de satiété.

Les ballons intra-gastriques connus comportent généralement au moins une poche souple apte à occuper une configuration repliée (ou à volume réduit) permettant l'implantation du ballon intra-gastrique par voie orale.

La poche est ensuite destinée à être remplie, une fois le ballon intra-gastrique implanté dans l'estomac, à l'aide d'un fluide de gonflage, et par exemple à l'aide d'air ou de liquide physiologique de manière à conférer au ballon intra-gastrique sa forme fonctionnelle, dans laquelle il occupe un volume suffisant au sein de l'estomac pour occuper une partie importante de l'espace disponible pour les aliments.

La poche est généralement réalisée à partir de matériaux présentant de bonnes propriétés d'étanchéité de manière à éviter la fuite progressive du fluide contenu dans la poche vers l'estomac et le dégonflage du ballon intra-gastrique. En particulier, l'étanchéité de la poche doit être suffisante pour que le ballon intra-gastrique conserve sa forme fonctionnelle pendant toute la durée du traitement qui peut varier de quelques semaines à plusieurs mois.

Dans les ballons intra-gastriques connus, la poche comporte un moyen de connexion, tel qu'une valve, destiné à permettre le raccordement du ballon intra-gastrique à une source de fluide de gonflage, par l'intermédiaire d'un organe de connexion comprenant par exemple un cathéter et une aiguille de gonflage. Le moyen de connexion comprend en outre des moyens assurant son étanchéité, une fois l'organe de connexion retiré, afin d'éviter la fuite du fluide contenu dans la poche.

Un exemple de ces ballons de l'art antérieur est décrit dans US-A-4 739 758.

Dans le cas où le fluide de gonflage est formé par un liquide, tel que du liquide physiologique, les moyens utilisés peuvent se présenter sous la forme d'une valve *« en bec de canard ».* Les valves en *« bec de canard »* sont conçues de manière à autoriser le passage du fluide sous pression de l'extérieur vers l'intérieur du ballon intra-gastrique et à interdire le passage du fluide dans le sens inverse. Ces valves permettent en règle générale d'obtenir de bons résultats avec les liquides, mais ne donnent généralement pas satisfaction lorsque le fluide de gonflage est formé par un gaz. En effet, l'étanchéité procurée par ce type de valves n'est alors pas satisfaisante. On préfère alors utiliser des valves du type *« septum »*, formées par une membrane plus ou moins épaisse en silicone, apte à être transpercée par l'aiguille de gonflage et présentant un caractère auto-cicatrisant.

Les ballons intra-gastriques équipés avec les valves du type *« septum »* sont de fabrication simple et donnent généralement satisfaction, mais présentent toutefois plusieurs inconvénients non négligeables.

Tout d'abord, en cas de stockage prolongé, un phénomène de vieillissement du silicone, formant le septum, peut apparaître. En particulier, le septum peut devenir sec, cassant et perdre son élasticité et ses propriétés d'étanchéité sous l'effet du vieillissement.

Par ailleurs, les ballons intra-gastriques sont parfois vendus au sein d'un kit comprenant une aiguille de gonflage pré-installée sur le ballon, l'aiguille de gonflage étant pré-positionnée, dans sa position fonctionnelle, de manière à traverser le septum et à déboucher dans la poche du ballon intra-gastrique.

De tels kits peuvent être stockés au sein de boîtiers stériles ou non stériles pendant une période prolongée avant d'être utilisés. Or, la présence prolongée de l'aiguille de gonflage à travers le septum peut conduire à la formation d'un orifice qui, si la durée de stockage est importante, peut persister après le retrait de l'aiguille, une fois le ballon intra-gastrique gonflé. Cet orifice constitue alors un passage préférentiel par lequel le fluide contenu dans le ballon intra-gastrique est susceptible de s'échapper, entraînant alors le dégonflage du ballon.

### EXPOSE DE L'INVENTION

Les objets assignés à l'invention visent en conséquence à porter remède aux différents inconvénients énumérés précédemment et à proposer un nouveau ballon intra-gastrique dont l'étanchéité est supérieure à celle des ballons intra-gastriques de l'art antérieur, même après une période de stockage prolongée.

Un autre objet de l'invention vise à proposer un nouveau ballon intra-gastrique susceptible d'être connecté facilement, de manière étanche et en toute sécurité à une source de fluide de gonflage.

Un autre objet de l'invention vise à proposer un nouveau ballon intra-gastrique qui soit particulièrement léger et bien supporté par le patient.

Un autre objet de l'invention vise à proposer un nouveau ballon intra-gastrique, dont l'extraction peut être réalisée facilement et rapidement, par voie orale.

Un autre objet de l'invention vise à proposer un nouveau ballon intra-gastrique susceptible d'être facilement et solidement agrippé par des outils d'extraction endoscopique.

Les objets assignés à l'invention visent également à proposer un nouveau kit comprenant un ballon intra-gastrique et un organe de connexion du ballon à une source de fluide de gonflage présentant, même après une longue période de stockage, de bonnes propriétés d'étanchéité.

Les objets assignés à l'invention sont atteints à l'aide d'un ballon intra gastrique destiné à être implanté à l'intérieur de l'estomac d'un patient pour réduire le volume de l'estomac dans le cadre d'un traitement de l'obésité, ledit ballon comprenant :
- au moins une poche apte à être remplie, au moins partiellement, avec un fluide de gonflage,
- un moyen de connexion, monté sur ladite poche et comportant un organe auto-obturant apte à être transpercé par un organe de connexion à une source de fluide de gonflage,
caractérisé en ce que l'organe auto-obturant comprend au moins deux membranes auto-cicatrisantes superposées, formant des membranes auto-cicatrisantes appariées, aptes à être successivement transpercées par l'organe de connexion, chaque membrane auto-cicatrisante étant montée de manière à venir en appui contre la membrane auto-cicatrisante appariée en vue de former une barrière étanche apte à refermer automatiquement le passage ménagé à travers la membrane auto-cicatrisante appariée une fois l'organe de connexion retiré.

Les objets assignés à l'invention sont également atteints à l'aide d'un kit de mise en forme d'un ballon intra-gastrique au sein de l'estomac d'un patient dans le cadre d'un traitement de l'obésité comprenant :
- un ballon intra-gastrique conforme à l'invention, au moins tel que décrit précédemment,
- un organe de connexion du ballon à la source de fluide de gonflage, monté, dans sa configuration de stockage, de manière à traverser de part en part les membranes auto-cicatrisantes superposées pour déboucher dans la poche.

### DESCRIPTIF SOMMAIRE DES DESSINS

D'autres objets et avantages de l'invention apparaîtront plus en détails à la lecture de la description qui suit, ainsi qu'à l'aide des dessins annexés ci-après, donnés à titre purement illustratif et non limitatif, dans lesquels :
- La figure 1 illustre, selon une vue en coupe, un ballon intra-gastrique conforme à l'invention, comportant une unique poche, avec un moyen de connexion muni d'un organe auto-obturant en train d'être transpercé par une aiguille de gonflage.
- La figure 2 illustre, selon une vue en coupe, une variante de réalisation d'un ballon intra-gastrique conforme à l'invention comportant deux poches concentriques.
- La figure 3 illustre, selon une vue en perspective, un moyen de connexion monté sur un ballon intra-gastrique conforme à l'invention.
- La figure 4 illustre, selon une vue en coupe suivant la ligne A-A illustrée sur la figure 3, le moyen de connexion illustré sur la figure 3.
- La figure 5 illustre, selon une vue schématique en coupe, un détail de l'organe auto-obturant illustré sur la figure 1.
- La figure 6 illustre, selon une vue schématique en coupe, le détail de la figure 5 après le retrait de l'aiguille de gonflage.
- La figure 7 illustre, selon une vue en coupe, une variante de réalisation d'un moyen de connexion conforme à l'invention.

### MEILLEURE MANIERE DE REALISER L'INVENTION

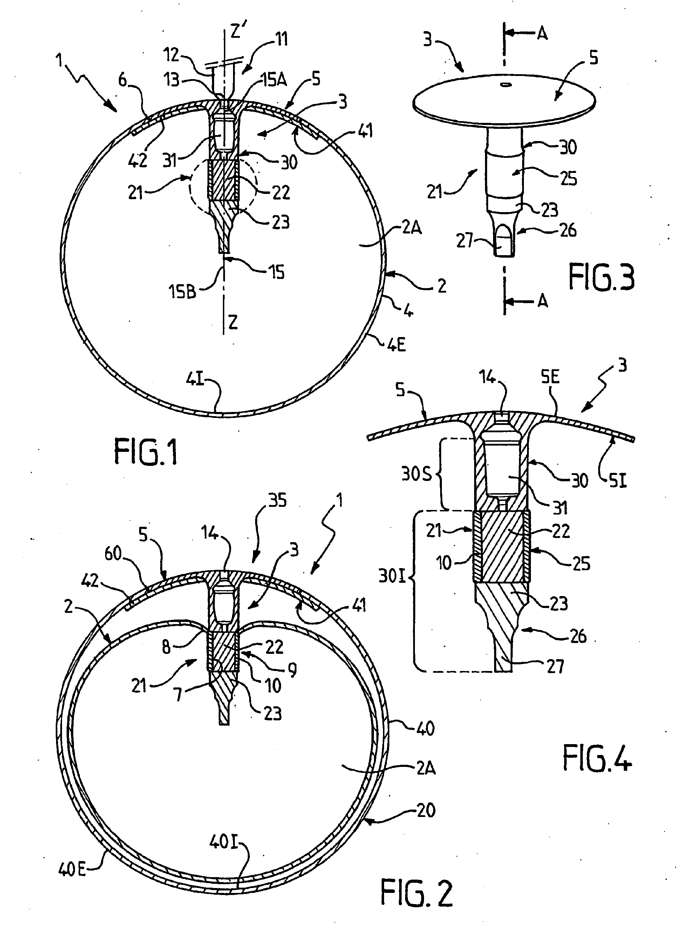

Les figures 1 et 2 illustrent- deux variantes de réalisation d'un ballon intra-gastrique 1 conforme à l'invention dans sa configuration fonctionnelle, c'est-à-dire expansée, dans laquelle il occupe une partie importante de l'espace disponible pour les aliments au sein de l'estomac.

Le ballon intra-gastrique 1 conforme à l'invention présente avantageusement un caractère expansible, c'est-à-dire qu'il est réalisé à partir de matériaux souples et de préférence à partir de matériaux élastomères tels que le silicone ou le polyuréthane thermoplastique élastomère, permettant son expansion entre une configuration repliée (non représentée) dans laquelle il occupe un faible volume permettant son implantation par voie orale, et d'autre part une configuration expansée dans laquelle il occupe un volume fonctionnel (figures 1 et 2).

Le ballon intra-gastrique 1 conforme à l'invention comprend au moins une poche 2 apte à être remplie, au moins partiellement, avec un fluide de gonflage et délimitant un volume interne 2A.

Le ballon intra-gastrique 1 conforme à l'invention comprend également un moyen de connexion 3, monté sur ladite poche 2 et destiné à être relié à une source de fluide (non représentée) à l'aide d'un organe de connexion 11 pour assurer l'expansion du ballon intra-gastrique 1 dans l'estomac par remplissage avec le fluide de gonflage.

Selon un premier mode de réalisation de l'invention illustré sur la figure 1, le ballon intra-gastrique 1 comprend une unique poche 2 formant une enveloppe superficielle 4 pour le ballon intra-gastrique 1. De façon préférentielle, la poche 2 est réalisée à partir d'un matériau souple biocompatible, et de préférence à partir de silicone de grade médical.

Selon ce premier mode de réalisation, le moyen de connexion 3 est monté sur la poche 2 et de préférence solidarisé avec cette dernière, par exemple par collage ou soudure. Le moyen de connexion 3 comporte à cet effet une collerette 5 destinée à permettre la fixation étanche du moyen de connexion 3 sur la poche 2, par exemple par collage ou soudure de la collerette 5 sur le pourtour d'une lumière 6 ménagée à travers la poche 2.

Selon un deuxième mode de réalisation illustré sur la figure 2, le ballon intra-gastrique 1 conforme à l'invention comprend au moins une première et une deuxième poches 2, 20 préférentiellement souples, la première poche 2 étant disposée à l'intérieur de la deuxième poche 20 de manière à former une première poche 2 interne et une deuxième poche 20 externe. Selon cette variante, le moyen de connexion 3 est fixé de façon étanche sur la première poche 2 et plus précisément monté au sein d'un passage 7 délimité par un col 8 s'étendant vers l'intérieur de la première poche 2. Plus précisément, le moyen de connexion 3 est fixé sur la première poche 2 à l'aide d'un élément de fixation 9 préférentiellement formé par une bague 10. L'élément de fixation 9 est ainsi avantageusement conformé et disposé pour exercer une pression suffisante sur le col 8 pour le pincer entre le moyen de connexion 3 et l'élément de fixation 7.

Selon ce deuxième mode de réalisation de l'invention, la première poche 2 forme un moyen de mise en forme de la deuxième poche 20, cette dernière formant alors l'enveloppe superficielle 40 du ballon intra-gastrique 1. Le fluide de gonflage et notamment l'air est avantageusement introduit dans la première poche 2 engendrant ainsi son gonflage. L'expansion de la première poche 2 engendre à son tour, à la manière d'une *« chambre à air »* l'expansion et la mise en forme de la deuxième poche 20 formant l'enveloppe superficielle 40 du ballon intra-gastrique 1.

Avantageusement, la première et la deuxième poches 2, 20 sont réalisées à partir de matériaux différents et non nécessairement compatibles.

L'expression *« non compatibles »* fait référence à des matériaux dont l'assemblage classique par collage ou par soudure s'avèrerait particulièrement difficile, voire impossible compte-tenu des contraintes de production industrielle et des exigences médicales en matière de qualité et de sécurité.

De façon préférentielle, les première et deuxième poches 2, 20 sont réalisées à partir de matériaux élastomères, la première poche 2 étant de préférence réalisée à partir d'un matériau à effet barrière aux gaz, tel que le polyuréthane thermoplastique élastomère et la deuxième poche 20 étant de préférence réalisée à partir d'un matériau biocompatible et présentant une bonne résistance mécanique, tel que le silicone. L'utilisation d'un matériau à effet barrière pour former la première poche 2 permet avantageusement de diminuer l'épaisseur de la première poche 2 tout en conservant, voire en améliorant l'étanchéité du ballon.

Selon ce deuxième mode de réalisation de l'invention, le pourtour de la collerette 5 est préférentiellement collé ou soudé avec la lumière 60 ménagée à travers la deuxième poche 20, le moyen de connexion 3 assurant ainsi l'obturation étanche de la deuxième poche 20, c'est-à-dire de l'enveloppe superficielle 40 du ballon intra-gastrique 1. La configuration à deux poches illustrée sur la figure 2 est préférée à la configuration à une poche illustrée sur la figure 1 car elle permet d'obtenir un ballon intra-gastrique 1 présentant une résistance mécanique et une étanchéité supérieures à, celles des ballons simple poche, tout en conservant un encombrement faible à l'état plié, grâce notamment à la faible épaisseur de la poche interne.

L'organe de connexion 11 comprend avantageusement un cathéter 12 destiné à assurer la liaison fluidique entre le ballon intra-gastrique 1 et la source de fluide de gonflage et conçu pour être raccordé au ballon intra-gastrique 1 à l'aide d'un embout 13 introduit au sein d'un logement 14 ménagé dans le moyen de connexion 3. L'organe de connexion 11 comprend également une aiguille de gonflage 15, préférentiellement formée par une aiguille creuse, dont l'une des extrémités 15A est solidarisée avec l'embout 13 et l'autre extrémité 15B, préférentiellement atraumatique, est située à l'intérieur du volume interne 2A. Tel que cela est illustré sur la figure 1, l'aiguille de gonflage est introduite à travers le moyen de connexion 3 suivant un axe de perforation Z-Z'.

Le moyen de connexion 3 comporte, selon l'invention, un organe auto-obturant 21 apte à être transpercé par l'organe de connexion 11 et plus précisément par l'aiguille de gonflage 15 (figure 1). Avantageusement, le moyen de connexion 3 comporte un corps principal 30 de préférence sensiblement allongé et s'étendant vers l'intérieur du ballon intra-gastrique 1. L'organe auto-obturant 21 est alors préférentiellement formé sur le corps principal 30 et donc situé à l'intérieur du ballon intra-gastrique 1, dans le volume interne 2A.

Selon l'invention, l'organe auto-obturant 21 comprend au moins deux membranes auto-cicatrisantes 22, 23 superposées formant des membranes auto-cicatrisantes 22, 23 appariées. Selon l'invention, les membranes auto-cicatrisantes 22, 23 sont conçues et disposées de manière à être successivement transpercées par l'organe de connexion 11 et notamment par l'aiguille de gonflage 15. En traversant de part en part les membrane auto-cicatrisantes 22, 23, l'aiguille de gonflage 15 ménage ainsi un passage 24 à travers chaque membrane.

L'expression « *membrane auto-cicatrisante* » fait référence à une membrane (ou paroi) en matériau suffisamment souple pour pouvoir être transpercée par l'organe de connexion 11, notamment par l'aiguille de gonflage 15, et présentant des propriétés d'élasticité et de mémoire de forme suffisantes pour combler le passage 24 laissé libre après le retrait de l'aiguille de gonflage 15, formant ainsi une membrane étanche.

Selon une caractéristique essentielle de l'invention, chaque membrane auto-cicatrisante 22, 23 est montée de manière à venir en appui contre la membrane auto-cicatrisante 22, 23 appariée en vue de former une barrière étanche apte à refermer automatiquement le passage 24 ménagé à travers la membrane auto-cicatrisante 22, 23 appariée une fois l'organe de connexion retiré. L'association des deux membranes auto-cicatrisantes 22, 23 conformes à l'invention confère ainsi à l'organe auto-obturant 21 ses propriétés d'auto-obturation, c'est-à-dire sa capacité à s'obturer automatiquement et sans intervention extérieure une fois l'organe de connexion 11 et plus précisément l'aiguille de gonflage 15 retirée.

Les propriétés d'auto-obturation de l'organe auto-obturant 21 sont obtenues pas la conjugaison de deux effets distincts, à savoir :
- le caractère auto-cicatrisant propre de chaque membrane auto-cicatrisante 22, 23, qui permet à ces dernières de s'auto-cicatriser et de se refermer indépendamment l'une de l'autre en comblant le passage 24 formé après le retrait de l'organe de connexion 11,
- l'action réciproque d'obturation exercée par chaque membrane auto-cicatrisante 22, 23 sur la membrane auto-cicatrisante 22, 23 appariée.

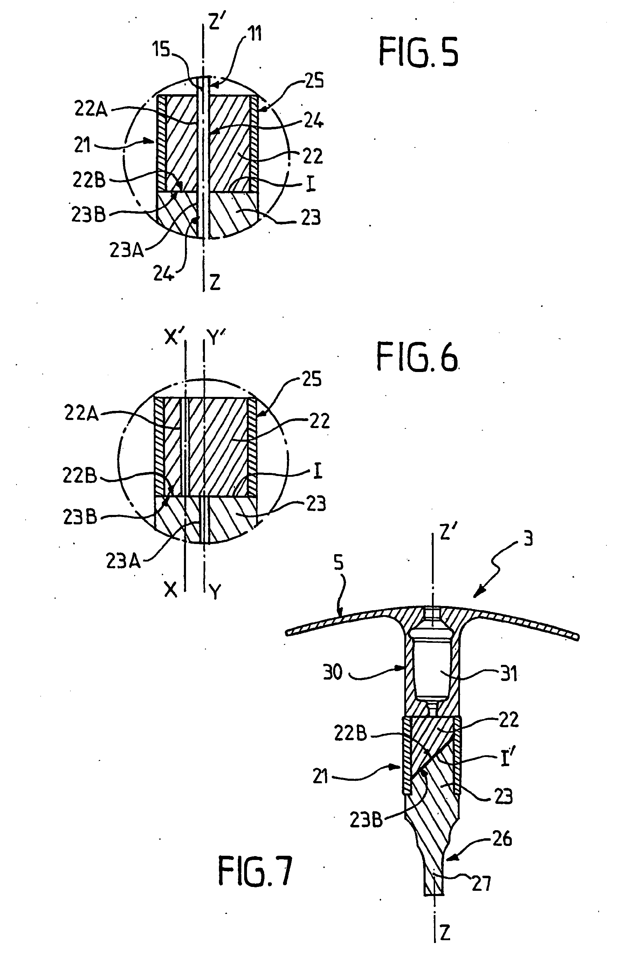

En effet, tel que cela est illustré sur la figure 5, le passage 24 ménagé à travers l'organe auto-obturant 21 par l'organe de connexion 11 et plus précisément par l'aiguille de gonflage 15 se décompose en deux sous-passages 22A, 23A ménagés respectivement dans une première et une deuxième membranes auto-cicatrisantes 22, 23 formant la paire de membranes susmentionnée.

Les deux sous-passages 22A, 23A ainsi ménagés, initialement alignés avec l'axe de perforation Z-Z' de l'aiguille de gonflage 15, ne demeurent pas en alignement axial l'un par rapport à l'autre une fois l'aiguille de gonflage 15 retirée. En d'autres termes, sous l'effet du retour élastique des membranes auto-cicatrisantes 22, 23 vers leur configuration initiale, autorisé par le retrait de l'aiguille de gonflage 15, les axes respectifs X-X' et Y-Y' selon lesquels s'étendent les sous-passages 22A, 23A vont se décaler latéralement, tel que cela est illustré sur la figure 6. Par le biais de ce décalage, le sous-passage 22A ménagé à travers la première membrane auto-cicatrisante 22 se trouve alors refermé par la deuxième membrane auto-cicatrisante 23 tandis que le sous-passage 23A ménagé à travers la deuxième membrane auto-cicatrisante 23 se trouve refermé par la première membrane auto-cicatrisante 22, chaque membrane auto-cicatrisante 22, 23 formant alors une barrière étanche à l'encontre du sous-passage 22A, 23A situé en regard.

De façon particulièrement avantageuse, les membranes auto-cicatrisantes 22, 23 viennent ainsi en appui étanche l'une contre l'autre pour assurer réciproquement la fermeture des sous-passages 22A, 23A. Les membranes auto-cicatrisantes 22, 23 ne sont pas nécessairement comprimées l'une contre l'autre, un simple appui passif pouvant suffire à assurer l'étanchéité à l'interface I entre les membranes auto-cicatrisantes 22, 23.

Il est bien évidemment envisageable, sans sortir du cadre de l'invention, que l'organe auto-obturant 21 comporte plus de deux membranes auto-cicatrisantes superposées, et par exemple trois ou quatre membranes auto-cicatrisantes.

L'association de deux membranes auto-cicatrisantes 22, 23 distinctes pour former l'organe auto-obturant 21 s'avère particulièrement avantageuse dans le cas où l'organe de connexion 11, et notamment l'aiguille de gonflage 15 est montée à travers l'organe auto-obturant 21 pendant une période prolongée, par exemple dans le cas d'un stockage de longue durée. Dans ce cas, on peut observer une diminution du pouvoir d'auto-cicatrisation propre à chaque membrane auto-cicatrisante 22, 23. Ce phénomène peut notamment provenir du vieillissement du matériau (silicone par exemple) formant les membranes, qui devient sec et cassant et perd ses propriétés d'élasticité. Le passage 24, et en particulier les sous-passages 22A, 23A sont alors susceptibles de se maintenir et de ne pas se cicatriser une fois l'aiguille de gonflage 15 retirée.

Dans les ballons intra-gastriques de l'art antérieur, pour lesquels l'organe auto-obturant 21 est formé par une membrane unique, le passage non cicatrisé ménagé à travers cette dernière constitue alors une source non négligeable de fuite du fluide de gonflage contenu dans le volume interne 2A. Au contraire, avec la structure de l'organe auto-obturant 21 conforme à l'invention, l'organe auto-obturant 21 conserve ses propriétés d'auto-obturation, même si les membranes auto-cicatrisantes 22, 23, en raison de la diminution de leur pouvoir d'auto-cicatrisation intrinsèque, ne sont plus en mesure d'assurer individuellement leur propre cicatrisation.

Certains paramètres peuvent contribuer à augmenter la probabilité statistique d'obtenir un décalage entre les sous-passages 22A, 23A après le retrait de l'aiguille de gonflage et à augmenter l'amplitude de ce décalage.

En particulier, la différence de dureté ou d'élasticité entre les membranes auto-cicatrisantes 22, 23 constitue un premier paramètre susceptible de favoriser le décalage des sous-passages 22A, 23A. En effet, si les membranes auto-cicatrisantes 22, 23 ne possèdent pas les mêmes propriétés d'élasticité et de dureté, leur comportement va être différent lors du retrait de l'aiguille de gonflage 15, ce qui va contribuer au décalage des sous-passages 22A, 23A l'un par rapport à l'autre.

D'une manière générale, un état au repos différent pour chaque membrane auto-cicatrisante 22, 23 pourra contribuer à décaler les sous-passages 22A, 23A une fois l'aiguille de gonflage 15 retirée. En particulier, chaque membrane auto-cicatrisante 22, 23 peut, en configuration de repos, c'est-à-dire avant son transpercement par l'aiguille de gonflage 15, se trouver dans un état relaxé ou dans un état comprimé. Suivant qu'elle se trouve dans un état relaxé ou comprimé, chaque membrane auto-cicatrisante 22, 23 ne va pas observer le même comportement une fois l'aiguille de gonflage retirée. Cette différence de comportement va alors augmenter la probabilité statistique d'observer un décalage entre les sous-passages 22A, 23A.

Avantageusement, les membranes auto-cicatrisantes 22, 23 sont formées à partir de matériaux à mémoire de forme présentant sensiblement les mêmes propriétés de souplesse et d'élasticité de manière à assurer réciproquement leur étanchéité. De façon préférentielle, les membranes auto-cicatrisantes 22, 23 sont réalisées à partir d'un matériau élastomère tel que le silicone. De façon encore plus préférentielle, les membranes auto-cicatrisantes sont réalisées à partir du même matériau, de préférence à partir de silicone, afin d'optimiser l'étanchéité à l'interface 1. On obtient ainsi; à l'interface 1, un contact silicone sur silicone présentant un caractère étanche.

Il est bien évidemment envisageable d'utiliser tout type de matériau, autre que le silicone, présentant des propriétés de souplesse, d'étanchéité et d'élasticité suffisantes pour pouvoir être transpercé par une aiguille et s'auto-cicatriser; une fois l'aiguille retirée.

Selon une variante de réalisation de l'invention, les membranes auto-cicatrisantes 22, 23 sont réalisées à partir de matériaux (par exemple à base de silicones) de duretés différentes.

Selon un premier mode de réalisation de l'invention, les membranes auto-cicatrisantes 22, 23 sont montées libres, au moins localement, l'une par rapport à l'autre de façon à permettre, au moins localement, le déplacement de l'une le long de l'autre. De façon préférentielle, les membranes auto-cicatrisantes 22, 23 sont montées libres l'une par rapport à l'autre au moins dans la zone de perforation par l'aiguille de gonflage 15. Un tel montage favorise ainsi le décalage des sous-passages 22A, 23A, une fois l'aiguille de gonflage retirée. Une telle configuration n'est cependant pas nécessaire pour obtenir le décalage.

Selon un deuxième mode de réalisation préférentielle de l'invention, les membranes auto-cicatrisantes 22, 23 sont attachées l'une à l'autre. La solidarisation des membranes auto-cicatrisantes 22, 23 l'une avec l'autre n'est en effet pas suffisante pour empêcher, à petite échelle, le décalage des sous-passages 22A, 23A. Ainsi, on observe la non coïncidence des sous-passages 22A, 23A après le retrait de l'organe de connexion 11 alors même que les membranes auto-cicatrisantes 22, 23 sont attachées l'une à l'autre.

Les membranes auto-cicatrisantes 22, 23 viennent avantageusement en appui l'une contre l'autre, au niveau de l'interface I, par l'intermédiaire d'au moins deux faces de contact 22B, 23B lesdites faces de contact 22B, 23B étant fixées l'une à l'autre, par exemple par collage ou soudure. De façon encore plus préférentielle, les faces de contact 22B, 23B sont fixées l'une à l'autre sur sensiblement toute leur surface.

Un autre mode de réalisation de l'organe auto-obturant 21, illustré sur la figure 7, contribue à augmenter la probabilité statistique d'obtenir un décalage entre les sous-passages 22A, 23A. Selon ce mode de réalisation, les deux membranes auto-cicatrisantes 22, 23 viennent en appui l'une contre l'autre au niveau d'une interface l'inclinée par rapport à l'axe de perforation Z-Z', c'est-à-dire non perpendiculaire à l'axe de perforation Z-Z'. Les faces de contact 22B, 23B sont alors inclinées par rapport à l'axe de perforation Z-Z' et forment un angle aigu avec ce dernier.

Avantageusement, et tel que cela est illustré aux figures, l'organe auto-obturant 21 comprend un organe de compression 25, du genre bague, disposé de manière à entourer au moins l'une des membranes auto-cicatrisantes 22, 23 de manière à la comprimer, de préférence latéralement et de manière centripète. L'organe de compression 25 et l'élément de fixation 9 sont préférentiellement formés par le même élément, à savoir la bague 10.

De façon préférentielle, la paire de membranes auto-cicatrisantes 22, 23 est formée par une première membrane auto-cicatrisante 22, comprimée par l'organe de compression 25 et une deuxième membrane auto-cicatrisante 23, non comprimée par l'organe de compression 25 et donc au repos, c'est-à-dire dans un état relaxé (figure 4). Les première et deuxième membranes auto-cicatrisantes 22, 23 ont alors un comportement différent après le retrait de l'organe de connexion 11, ce qui favorise le décalage des sous-passages 22A, 23A.

Par ailleurs, le recours à un organe de compression 25 permet également d'améliorer le pouvoir auto-cicatrisant propre de la première membrane auto-cicatrisante 22.

Dans la configuration dite verticale du ballon intra-gastrique 1, illustrée sur les figures 1 et 2, le moyen de connexion 3 et le corps principal 30 s'étendent suivant une direction sensiblement verticale, coïncidant avec l'axe de perforation Z-Z'. Dans cette configuration verticale, la première membrane auto-cicatrisante 22, comprimée, est préférentiellement située au-dessus de la deuxième membrane auto-cicatrisante 23, relaxée.

Avantageusement, le corps principal 30 comprend une portion supérieure 30S, évidée de manière à former une cavité 31, par exemple cylindrique, et une portion inférieure 301, comprenant l'organe auto-obturant 21.

Il est intéressant de noter que la présence de la cavité 31 au sein du moyen de connexion 3, et notamment au sein du corps principal 30 est indépendante de la présence ou non des deux membranes auto-cicatrisantes 22, 23. La cavité 31 vise en effet simplement à faciliter la préhension du ballon intra-gastrique 1 lors de son extraction, et pourrait donc être associée à tout type d'organe auto-obturant, et par exemple à une simple membrane auto-cicatrisante.

Afin de favoriser la préhension du ballon intra-gastrique, la portion supérieure 30S est ainsi évidée sur une distance suffisante pour que la paroi (ou les parois) du corps principal 30 délimitant la cavité 31 puisse se rabattre sur elle-même lors du pincement de la portion supérieure 30S du corps principal 30 avec un outil d'extraction (non représenté) du ballon intra-gastrique 1, tel qu'une pince endoscopique.

De façon préférentielle, la cavité 31 s'étend longitudinalement, selon l'axe de perforation Z-Z', sur une longueur supérieure à sa largeur moyenne (ou à son diamètre dans le cas d'une cavité cylindrique) mesurée suivant une direction sensiblement perpendiculaire à l'axe de perforation Z-Z'.

A titre d'exemple illustratif et non limitatif, la cavité 31 pourra présenter une longueur supérieure à 1,5 fois sa largeur moyenne.

La longueur de la cavité 31 résulte avantageusement d'un compromis entre :
- d'une part la nécessité de limiter la longueur totale du moyen de connexion 3 et du corps principal 30 pour ne pas gêner l'implantation du ballon intra-gastrique 1 par voie orale,
- et d'autre part la nécessité de pouvoir pincer le moyen de connexion 3 à proximité de l'enveloppe superficielle 4, 40 du ballon intra-gastrique 1 pour permettre l'extraction de ce dernier.

Ainsi, l'implantation du ballon intra-gastrique 1 s'effectue alors que l'aiguille de gonflage 15 est montée sur le moyen de connexion 3 de manière à traverser longitudinalement le corps principal 30. Le corps principal 30 et l'aiguille de gonflage 15 forment alors un ensemble rigide présentant une flexibilité longitudinale très faible. Or, le chirurgien, pour introduire le ballon intra-gastrique 1 dans les voies orales du patient doit pouvoir bénéficier d'une certaine souplesse, d'où la nécessité de réduire au minimum la longueur du corps principal 30.

De façon préférentielle, la collerette 5 s'étend radialement à la surface du ballon intra-gastrique 1 de manière à former une zone de préhension 35 du ballon intra-gastrique 1 par l'outil d'extraction (non représenté).

Il est envisageable de réaliser la collerette 5 dans un matériau présentant une dureté supérieure au matériau formant l'enveloppe superficielle 4, 40, par exemple à partir d'un silicone dur, de manière à offrir, au moins localement, une résistance mécanique supérieure et à éviter son transpercement par l'outil d'extraction. Il est néanmoins préférable, pour permettre le pliage du ballon intra-gastrique 1, de réaliser la collerette 5 à partir d'un matériau souple de dureté équivalente ou à peine supérieure à celle de l'enveloppe superficielle 4, 40.

Tel que cela est illustré sur les figures 1 et 2, la zone de préhension 35 du ballon intra-gastrique 1 est préférentiellement associée à un renfort 41 pour former une zone de préhension 35 renforcée dédiée à l'extraction du ballon intra-gastrique 1. Le renfort 41 se présente avantageusement sous la forme d'une membrane flexible 42 qui s'étend le long de la zone de préhension 35 de telle manière qu'elle ne fait pas saillie hors de l'enveloppe superficielle 4, 40 et ne forme donc aucune protubérance susceptible de porter atteinte à la géométrie régulière et au caractère atraumatique du ballon intra-gastrique 1.

De façon préférentielle, la forme du renfort 41 épouse sensiblement la forme de la collerette 5 et de l'enveloppe superficielle 4, 40 auxquelles il est associé.

De façon encore plus préférentielle, le renfort 41 est superposé à la collerette 5 à l'intérieur du ballon intra-gastrique 1 et préférentiellement collé ou soudé, sur toute sa surface, sur la surface interne 5l de la collerette 5. Le renfort 41 peut également se prolonger, tel que cela est illustré sur la figure 2, sous l'enveloppe superficielle 4, 40, le renfort 41 étant alors solidarisé à ladite enveloppe superficielle 4, 40 par soudure ou collage avec la face interne 41, 401 de l'enveloppe superficielle 4, 40. Il est toutefois bien évidemment envisageable, sans sortir du cadre de l'invention, de rapporter le renfort 41 sur la surface externe 5E de la collerette 5 ou même encore de noyer le renfort 41 dans l'épaisseur de la collerette 5. De la même façon, le renfort 41 pourra s'étendre sur la face externe 4E, 40E de l'enveloppe superficielle 4, 40 ou être noyé dans l'épaisseur de cette dernière.

Le renfort 41 comprend préférentiellement au moins une pièce textile, comprenant par exemple un tulle de polyester, un matériau tissé (ou non tissé) réalisé à partir de fibres de polyamide, et/ou de fibres d'aramide. On peut également envisager de mettre en oeuvre, en tant que pièce de renfort, une structure fibreuse de type « *nid d'abeilles »* bien connue en tant que telle.

L'organe auto-obturant 21 comprend avantageusement un organe d'étirement 26 apte à être manipulé pour assurer l'étirement axial de l'organe auto-obturant 21, notamment suivant une direction principale d'extension parallèle à l'axe de perforation Z-Z', et permettre le montage de l'organe de compression 25 autour d'au moins l'une des membranes auto-cicatrisantes 22, 23. Ainsi, l'organe de compression 25 présente avantageusement un diamètre interne inférieur au diamètre externe au repos de la membrane auto-cicatrisante 22 à laquelle il est associé, de manière à pouvoir comprimer radialement et de manière centripète cette dernière.

L'étirement axial de la membrane auto-cicatrisante 22 à laquelle est associé l'organe de compression 25 permet alors de faire diminuer le diamètre externe de la membrane auto-cicatrisante 22 jusqu'à ce que ce dernier atteigne le diamètre interne de l'organe de compression 25 et notamment de la bague 10. Il est alors possible d'emmancher la bague 10 sur la membrane auto-cicatrisante 22 tel que cela est illustré sur la figure 4.

De façon préférentielle, l'organe d'étirement 26 est formé par une languette de traction 27 fixée sur ou venue de matière avec l'organe auto-obturant 21, et plus préférentiellement avec la deuxième membrane auto-cicatrisante 23, située sous la première membrane auto-cicatrisante 22. La languette de traction 27 forme ainsi avantageusement un prolongement du corps principal 30.

L'invention concerne également un kit de mise en forme d'un ballon intra-gastrique 1 destiné à être implanté au sein de l'estomac d'un patient dans le cadre d'un traitement de l'obésité.

Selon l'invention, le kit comprend un ballon intra-gastrique 1 conforme à l'invention et un organe de connexion 11 du ballon intra-gastrique 1 à la source de fluide de gonflage, monté, dans sa configuration de stockage, de manière à traverser de part en part les membranes auto-cicatrisantes 22, 23 superposées pour déboucher dans la poche 2. '

L'organe de connexion 11 comprend avantageusement une aiguille de gonflage 15 préférentiellement formée par une aiguille creuse apte à transpercer l'organe auto-obturant 21 et les membranes auto-cicatrisantes 22, 23 de manière à déboucher dans la poche 2.

La conception du ballon intra-gastrique 1 conforme à l'invention permet ainsi d'assurer, en toute sécurité, le remplissage du ballon intra-gastrique 1 une fois ce dernier disposé à l'intérieur de l'estomac du patient, sans risque de fuite et ce même après une durée de stockage prolongée du ballon intra-gastrique 1 avec l'aiguille de gonflage 15 pré-installée sur ce dernier.

Un autre avantage du ballon intra-gastrique 1 conforme à l'invention provient de sa facitité d'extraction liée à la présence de la cavité 31.

### POSSIBILITE D'APPLICATION INDUSTRIELLE

L'invention trouve son application industrielle dans la fabrication et l'utilisation de ballons intra-gastriques de traitement de l'obésité.

## Revendications

1. - Ballon intra-gastrique destiné à être implanté à l'intérieur de l'estomac d'un patient pour réduire le volume de l'estomac dans le cadre d'un traitement de l'obésité, ledit ballon intra-gastrique (1) comprenant :
- au moins une poche (2) apte à être remplie, au moins partiellement, avec un fluide de gonflage,
- un moyen de connexion (3), monté sur ladite poche (2) et comportant un organe auto-obturant (21) apte à être transpercé par un organe de connexion (11) à une source de fluide de gonflage,
**caractérisé en ce que** l'organe auto-obturant (21) comprend au moins deux membranes auto-cicatrisantes (22, 23) superposées, formant des membranes auto-cicatrisantes (22, 23) appariées, aptes à être successivement transpercées par l'organe de connexion (11), chaque membrane auto-cicatrisante (22, 23) étant montée de manière à venir en appui contre la membrane auto-cicatrisante (22, 23) appariée en vue de former une barrière étanche apte à refermer automatiquement le passage (24) ménagé à travers la membrane auto-cicatrisante (22, 23) appariée une fois l'organe de connexion (11) retiré.

2. - Ballon intra-gastrique selon la revendication 1 **caractérisé en ce que** les membranes auto-cicatrisantes (22, 23) sont formées à partir de matériaux à mémoire de forme présentant sensiblement les mêmes propriétés de souplesse et d'élasticité de manière à assurer réciproquement leur étanchéité.

3. - Ballon intra-gastrique selon l'une des revendications précédentes **caractérisé en ce que** les membranes auto-cicatrisantes (22, 23) sont réalisées à partir du même matériau, de préférence à partir de silicone.

4. - Ballon intra-gastrique selon la revendication 1 **caractérisé en ce que** les membranes auto-cicatrisantes (22, 23) sont réalisées à partir de matériaux de duretés différentes.

5. - Ballon intra-gastrique selon l'une des revendications précédentes **caractérisé en ce que** les membranes auto-cicatrisantes (22, 23) sont montées libres au moins localement l'une part rapport à l'autre de façon à permettre, au moins localement, le déplacement de l'une le long de l'autre.

6. - Ballon intra-gastrique selon l'une des revendications précédentes **caractérisé en ce que** les membranes auto-cicatrisantes (22, 23) sont attachées l'une à l'autre.

7. - Ballon intra-gastrique selon la revendication 6 **caractérisé en ce que** les membranes auto-cicatrisantes (22, 23) viennent en appui l'une contre l'autre par l'intermédiaire d'au moins deux faces de contact (22B, 23B), lesdites faces de contact (22B, 23B) étant fixées l'une à l'autre, par exemple par collage ou soudure.

8. - Ballon intra-gastrique selon la revendication 7 **caractérisé en ce que** les faces de contact (22B, 23B) sont fixées l'une à l'autre sur sensiblement toute leur surface.

9. - Ballon intra-gastrique selon l'une quelconque des revendications précédentes **caractérisé en ce que** l'organe auto-obturant (21) comprend un organe de compression (25), du genre bague, disposé de manière à entourer au moins l'une des membranes auto-cicatrisantes (22, 23) de manière à la comprimer.

10. -Ballon intra-gastrique selon la revendication 9 **caractérisé en ce que** la paire de membranes auto-cicatrisantes (22, 23) est formée par une première membrane auto-cicatrisante (22), comprimée par l'organe de compression (25) et une deuxième membrane auto-cicatrisante (23) non comprimée par l'organe de compression (25).

11. - Ballon intra-gastrique selon la revendication 9 ou 10 **caractérisé en ce que** l'organe auto-obturant (21) comprend un organe d'étirement (26), apte à être manipulé pour assurer l'étirement axial de l'organe auto-obturant (21), et permettre le montage de l'organe de compression (25) autour d'au moins l'une des membranes auto-cicatrisantes (22, 23).

12. -Ballon intra-gastrique selon la revendication 11 **caractérisé en ce que** l'organe d'étirement (26) est formé par une languette de traction (27), fixée sur ou venue de matière avec l'une des membranes auto-cicatrisantes (22, 23).

13. -Ballon intra-gastrique selon l'une des revendications précédentes **caractérisé en ce que** le moyen de connexion (3) comprend un corps principal (30), faisant saillie vers l'intérieur du ballon intra-gastrique (1), ledit corps principal (30) comprenant une portion supérieure (30S) évidée, de manière à former une cavité (31), et une portion inférieure (301), comprenant l'organe auto-obturant (21).

14. -Ballon intra-gastrique selon la revendication 13 **caractérisé en ce que** la portion supérieure (30S) est évidée sur une distance suffisante pour que la paroi du corps principal (30) délimitant la cavité (31) puisse se rabattre sur elle-même lors du pincement de la portion supérieure (30S) du corps principal (30) avec un outil d'extraction du ballon intra-gastrique (1), tel qu'une pince endoscopique.

15. - Ballon intra-gastrique selon l'une des revendications précédentes **caractérisé en ce que** le moyen de connexion (3) comprend une collerette (5) s'étendant radialement à la surface du ballon intra-gastrique (1), de manière à former une zone de préhension (35) du ballon intra-gastrique (1) par l'outil d'extraction.

16. - Ballon intra-gastrique selon la revendication 15 **caractérisé en ce que** la zone de préhension (35) est associée à un renfort (41), pour former une zone de préhension (35) renforcée dédiée à l'extraction du ballon intra-gastrique (1) hors de l'estomac.

17. -Kit de mise en forme d'un ballon intra-gastrique (1) destiné à être implanté au sein de l'estomac d'un patient dans le cadre d'un traitement de l'obésité comprenant :
- un ballon intra-gastrique (1) selon l'une des revendications 1 à 16,
- un organe de connexion (11) du ballon intra-gastrique (1) à la source de fluide de gonflage, monté, dans sa configuration de stockage, de manière à traverser de part en part les membranes auto-cicatrisantes (22, 23) superposées pour déboucher dans la poche (2).

18. -Kit selon la revendication 17 **caractérisé en ce que** l'organe de connexion (11) comprend une aiguille de gonflage (15), apte à transpercer les membranes auto-cicatrisantes (22, 23).

## Claims

1. Intra-gastric balloon intended to be implanted inside the stomach of a patient in order to reduce the volume of the stomach in the context of treatment for obesity, the said intra-gastric balloon (1) comprising:
- at least one pouch (2) able to be at least partially filled with an inflation fluid,
- a connection means (3) mounted on the said pouch (2) and comprising a self-closing member (21) able to be pierced by a member (11) for connection to an inflation fluid source,
**characterised in that** the self-closing member (21) comprises at least two superimposed self-healing membranes (22, 23) forming matched self-healing membranes (22, 23) able to be successively pierced by the connection member (11), each self-healing membrane (22, 23) being mounted so as to come into abutment against the matched self-healing membrane (22, 23) with a view to forming a sealed barrier able to automatically close the passage (24) formed through the matched self-healing membrane (22, 23) once the connection member (11) has been withdrawn.

2. intra-gastric balloon according to claim 1, **characterised in that** the self-healing membranes (22, 23) are formed by shape-memory materials having substantially the same properties of flexibility and elasticity so as to mutually provide their impermeability.

3. Intra-gastric balloon according to one of the preceding claims, **characterised in that** the self-healing membranes (22, 23) are produced from the same material, preferably from silicone.

4. intra-gastric balloon according to claim 1, **characterised in that** the self-healing membranes (22, 23) are produced from materials with different hardnesses.

5. Intra-gastric balloon according to one of the preceding claims, **characterised in that** the self-healing membranes (22, 23) are mounted so as to be free at least locally with respect to each other so as to allow, at least locally, the movement of one along the other.

6. intra-gastric balloon according to one of the preceding claims, **characterised in that** the self-healing membranes (22, 23) are attached to each other.

7. intra-gastric balloon according to claim 6, **characterised in that** the self-healing membranes (22, 23) come into abutment against each other by means of at least two contact faces (228, 23B), the said contact faces (22B, 23B) being fixed to each other, for example by adhesive bonding or welding.

8. Intra-gastric balloon according to claim 7, **characterised in that** the contact faces (22B, 23B) are fixed to each other over substantially their entire surface.

9. intra-gastric balloon according to any one of the preceding claims, **characterised in that** the self-closing member (21) comprises a compression member (25), of the ring type, disposed so as to surround at least one of the self-healing membranes (22, 23) so as to compress it.

10. intra-gastric balloon according to claim 9, **characterised in that** the pair of self-healing membranes (22, 23) is formed by a first self-healing membrane (22) compressed by the compression member (25) and a second self-healing membrane (23) not compressed by the compression member (25).

11. intra-gastric balloon according to claim 9 or 10, **characterised in that** the self-closing member (21) comprises a stretching member (26) able to be manipulated so as to provide the axial stretching of the self-closing member (21) and allow the mounting of the compression member (25) around at least one of the self-healing membranes (22, 23).

12. Intra-gastric balloon according to claim 11, **characterised in that** the stretching member (26) is formed by a traction tongue (27) fixed to or made in one piece with one of the self-healing membranes (22, 23).

13. Intra-gastric balloon according to one of the preceding claims, **characterised in that** the connection means (3) comprises a main body (30) projecting towards the inside of the intra-gastric balloon (1), the said main body (30) comprising a top portion (30S) hollowed out so as to form a cavity (31), and a bottom portion (30l) comprising the self-closing member (21).

14. Intra-gastric balloon according to claim 13, **characterised in that** the top portion (30S) is hollowed out over a sufficient distance so that the wall of the main body (30) delimiting the cavity (31) can fold back on itself when the top portion (30S) of the main body (30) is gripped with a tool for extracting the intra-gastric balloon (1), such as an endoscopic clamp.

15. Intra-gastric balloon according to one of the preceding claims, **characterised in that** the connection means (3) comprises a collar extending radially to the surface of the intra-gastric balloon (1), so as to form a gripping zone (35) for the intra-gastric balloon (1) to be gripped by the extraction tool.

16. intra-gastric balloon according to claim 15, **characterised in that** the gripping zone (35) is associated with a reinforcement (41) to form a reinforced gripping zone (35) dedicated to the extraction of the intra-gastric balloon (1) out of the stomach.

17. Kit for forming an intra-gastric balloon (1) intended to be implanted within the stomach of a patient in the context of obesity treatment, comprising:
- an intra-gastric balloon (1) according to one of claims 1 to 16,
- a connection member (11) for connecting the intra-gastric balloon (1) to the inflation fluid source, mounted, in its storage configuration, so as to pass right through the self healing membranes (22, 23) superimposed in order to emerge in the pouch (2).

18. Kit according to claim 17, **characterised in that** the connection member (11) comprises an inflation needle (15) able to pierce the self-healing membranes (22, 23).

## Patentansprüche

1. - Intragastrischer Ballon, der dazu vorgesehen ist, in das Innere des Magens eines Patienten implantiert zu werden, um das Volumen des Magens im Rahmen einer Fettleibigkeitsbehandlung zu reduzieren, wobei der intragastrische Ballon (1) Folgendes umfasst:
- mindestens eine Tasche (2), die dazu vorgesehen ist, mindestens teilweise mit einem Aufblasfluid gefüllt zu werden,
- ein Verbindungsmittel (3), das auf die Tasche (2) montiert ist und ein selbstschließendes Element (21) umfasst, das dazu geeignet ist, von einem Verbindungselement (11) mit einer Aufblasfluid-Quelle durchbohrt zu werden,
**dadurch gekennzeichnet, dass** das selbstschließende Element (21) mindestens zwei übereinander liegende selbstheilende Membranen (22, 23) umfasst, die gepaarte selbstheilende Membranen (22, 23) bilden, welche dazu geeignet sind, aufeinander folgend von dem Verbindungselement (11) durchbohrt zu werden, wobei jede selbstheilende Membran (22, 23) so angebracht ist, dass sie sich gegen die gepaarte selbstheilende Membran (22, 23) stützt, um eine dichte Barriere zu bilden, die dazu geeignet ist, den Durchgang (24) automatisch zu schließen, der durch die gepaarte selbstheilende Membran (22, 23) angeordnet ist, nachdem das Verbindungselement (11) zurückgezogen wurde.

2. - Intragastrischer Ballon nach Anspruch 1, **dadurch gekennzeichnet, dass** die selbstheilenden Membranen (22, 23) aus FormgedächtnismaLeriali.en gebildet sind, die im wesentlichen dieselben Eigenschaften der Flexibilität und der Elastizität aufweisen, um gegenseitig ihre Dichtigkeit sicherzustellen.

3. - Intragastrischer Ballon nach einem der vorhergehenden Ansprüche, **dadurch gekennzeichnet, dass** die selbstheilenden Membranen (22, 23) aus demselben Material hergestellt sind, vorzugsweise aus Silikon.

4. - Intragastrischer Ballon nach Anspruch 1, **dadurch gekennzeichnet, dass** die selbstheilende Membranen (22, 23) aus Materialien mit verschiedenen Härten hergestellt sind.

5. - Intragasbrischer Ballon nach einem der vorhergehenden Ansprüche, **dadurch gekennzeichnet, dass** die selbstheilenden Membranen (22, 23) mindestens lokal frei in Bezug zueinander montiert sind, um mindestens lokal die Verschiebung der einen entlang der anderen zu ermöglichen.

6. - Intragastrischer Ballon nach einem der vorhergehenden Ansprüche, **dadurch gekennzeichnet, dass** die selbstheilenden Membranen (22, 23) aneinander befestigt sind.

7. - Tntragastrischer Ballon nach Anspruch 6, **dadurch gekennzeichnet, dass** stich die selbstheilenden Membranen (22, 23) mithilfe von mindestens zwei Kontaktflächen (22B, 23B) gegeneinander stützen, wobei die Kontaktflächen (22B, 23B) aneinander befestigt sind, z.B. durch Kleben oder Schweißen.

8. - IntragasLrischer Ballon nach Anspruch 7, **dadurch gekennzeichnet, dass** die Kontaktflächen (22B, 23B) im Wesentlichen auf ihrer gesamten Oberfläche aneinander befestigt sind.

9. - Intragastrischer Ballon nach einem der vorhergehenden Ansprüche, **dadurch gekennzeichnet, dass** das selbstschließende Element (21) ein Kompressionselement (25) der Art eines Rings umfasst, das so angeordnet ist, dass es mindestens eine der selbstheilenden Membranen (22, 23) umgibt, um sie zusammenzudrücken.

10. - Intragastrischer Ballon nach Anspruch 9, **dadurch gekennzeichnet, dass** das Paar selbstheilender Membranen (22, 23) aus einer ersten selbstheilenden Membran (22) gebildet ist, die durch das Kompressionselement (25) zusammengedrückt wird, und einer zweiten selbstheilenden Membran (23), die nicht durch das Kompressionselement (25) zusammengedrückt wird.

11. Intragastrischer Ballon nach Anspruch 9 oder 10, **dadurch gekennzeichnet, dass** das selbstsahließende Element (21) ein Dehnungselement (26) umfasst, das dazu geeignet ist, manipuliert zu werden, um die axiale Dehnung des selbstschließenden Elementes (21) sicherzustellen und das Anbringen des Kompressionselementes (25) um mindestens eine der selbstheilenden Membranen (22, 23) zu ermöglichen.

12. - Intragastrischer Ballon nach Anspruch 11, **dadurch gekennzeichnet, dass** das Dehnungselement (26) aus einer Zuglasche (27) gebildet ist, die auf einer der selbstheilenden Membranen (22, 23) befestigt oder damit einstückig ist.

13. . Intragastrischer Ballon nach einem der vorhergehenden Ansprüche, **dadurch gekennzeichnet, dass** das Verbindungsmittel (3) einen Hauptkörper (30) umfasst, der in das Innere des intragastri.schen Ballons (1) hervorragt, wobei der Hauptkörper (30) einen ausgehöhlten oberen Teil (30S) umfasst, um einen Hohlraum (31) zu bilden, und einen unteren Teil (301), der das selbstschließende Element (21) umfasst.

14. - Intragastrischer Ballon nach Anspruch 13, **dadurch gekennzeichnet, dass** der obere Teil (30s) auf einer Strecke ausgehöhlt ist, die ausreichend ist, damit die Wand des Hauptkörpers (30), die den Hohlraum (31) begrenzt, beim Durchstechen des oberen Teils (30S) des Hauptkörpers (30) mit einem Werkzeug zum Entfernen des intragastrischen Ballons (1) wie z.B. einer endoskopischen Zange, auf sich selbst umklappen kann.

15. . Intragastrischer Ballon nach einem der vorhergehenden Ansprüche, **dadurch gekennzeichnet, dass** das Verbindungsmittel (3) einen Kragen (5) umfasst, der sich radial auf der Oberfläche des intragastrischen Ballons (1) erstreckt, um einen Greifbereich (35) des intragastrischen Ballons (1) für das Werkzeug zum Entfernen zu bilden.

16. . Intragastrischer Ballon nach Anspruch 15, **dadurch gekennzeichnet, dass** der Greifbereich (35) mit einer Verstärkung (41) verbunden ist, um einen verstärkten Greifbereich (35) zu bilden, der zum Entfernen des intragastrischen Ballons (1) aus dem Magen dient.

17. - Kit zum Formen eines intragastrischen Ballons (1), der dazu vorgesehen ist, im Rahmen einer FettleibigkeitsBehandlung in den Magen eines Patienten implantiert zu werden, umfassend:
- einen intragastrischen Ballon (1) nach einem der Ansprüche 1 bis 16,
- ein Verbindungselement (11) des intragastrischen Ballons (1) mit der Aufblasfluid-Quelle, das in seiner Lagerkonfi.guration so angeordnet ist, dass es die aufeinander liegenden selbstheilenden Membranen (22, 23) vollständig durchquert, um in der Tasche (2) zu münden.

18. - Kit nach Anspruch 17, **dadurch gekennzeichnet, dass** das Verbindungselement (11) eine Aufblasnadel (15) umfasst, die dazu geeignet ist, die selbstheilenden Membranen (22, 23) zu durchbohren.
